# EUROPEAN PATENT SPECIFICATION

(11) **EP 1 296 928 B1**
(45) Date of publication and mention of the grant of the patent: **19.11.2008**
(21) Application number: 01951522.0
(22) Date of filing: 25.05.2001
(51) Int. Cl.: C07C 69/00, C07C 67/52

(54) **A PROCESS FOR THE PURIFICATION OF DIACEREIN**
VERFAHREN ZUR REINIGUNG VON DIACEREIN
PROCEDE DE PURIFICATION DE DIACEREINE

(30) Priority: 13.06.2000 IT MI20001317
(43) Date of publication of application: 02.04.2003
(73) Proprietor: Synteco S.p.A., 27028 S. Martino Siccomario (IT)
(72) Inventor: MAGGI, Domenico, I-27028 San Martino Siccomario (IT)
(74) Representative: Minoja, Fabrizio
(86) International application number: PCT/EP2001/006019
(87) International publication number: WO 2001/096276

(56) References cited:
- EP-A- 0 636 602
- EP-A- 0 928 781
- WO-A-00/68179
- US-A- 5 652 265

## Description

The present invention relates to an improved process for the purification of diacerein.

The most known process for the preparation of diacerein comprises the acetylation of aloin with formation of acetylaloin (acetylbarbaloin) and then its subsequent oxidation with chromic acid.

The subsequent purification process aims at obtaining a product substantially free from impurities and, more particularly, at removing aloe-emodin, which is known to have mutagenic properties above threshold values of 70 p.p.m., as well as at obtaining a product having chromium content below 50 p.p.m.

A number of methods for the purification of crude diacerein are known. EP-A- 96900687 discloses a process wherein diacerein is suspended in a mixture of organic solvents and water, a tertiary amine is added to obtain a solution, diacerein is salified by addition of an alkali/alkaline earth metal salt with an organic acid and finally the resulting aqueous solution of the diacerein salt is hydrolyzed in slightly acidic medium. It has however been observed that dissolution of the diacerein salt in water, before acidification with weak acids, induces partial deacetylation with consequent formation of one/two impurities and loss of the product titre (i.e. of the purity) of the order of 0.5 a 5%. Said loss is statistically about 2%.

On the other hand, it is known from chemical literature that the protection of hydroxyls by acetylation is reversible, due to hydrolysis in even slightly acidic or basic medium. In the case of diacerein, this is stable in medium from neutral to acidic, whereas it rapidly undergoes deacetylation in even slightly basic medium: in particular, basicity induced by the diacerein sodium salt is sufficient to cause deacetylation. Any subsequent attempts at reacetylation with acetic acid, acetic anhydride or acetyl chloride are complex, incomplete and involve loss of yield.

EP 636,602 discloses a multi-step purification process which is very complex and makes use of toxic solvents. More particularly, crude diacerein is dried and purified first by dissolution in methylene chloride and triethylamine, then by precipitation through addition of acids. The product is separated by centrifugation, dried and crystallized first with methylcellosolve, filtered, dried and recrystallized with N,N-dimethylacetamide, filtered again and repeatedly washed with water to remove the solvent, which is otherwise difficult to remove by drying.

International Patent number WO 00/68719, in the Applicant's name, discloses a process for the preparation of highly pure diacerein by simple crystallization of crude diacerein from either an acetic anhydride/acetic acid mixture, or acetic anhydride alone, in the presence of small amounts of ethylenediaminetetraacetic acid (EDTA) to remove any traces of chromium from the previous chromic oxidation of acetylbarbaloin, said crystallization being followed by dissolution in an acetone/triethylamine mixture and reprecipitation with diluted phosphoric acid, in order to completely remove acetic acid. According to said patent application, crude diacerein is hot dissolved in 60-100 parts (by volume/weight) of an acetic anhydride/acetic acid mixture in a volume/volume ratio ranging from 10:90 to 30:70; or in 10-25 volumes of acetic anhydride alone. The hot solution is added with 0.05 to 0.2 % by weight of EDTA, compared with diacerein, and the EDTA-chromium complex is filtered off while hot. After cooling, the resulting precipitate is filtered or centrifuged, washed with acetic acid, then with water, dissolved in 10-20 parts by volume of acetone/triethylamine in a ratio ranging from 25:1 to 35:1, then reprecipitated with a 4-8% phosphoric acid aqueous solution. After washing with water and drying, diacerein is obtained in 80-90% yields, with minimum purity 99.7%, containing less than 70 p.p.m. of aloe-emodin and not more than 15 p.p.m. of chromium, determined by atomic absorption.

It has now surprisingly found, and it is the object of the invention, that highly pure diacerein (purity not below 99.8%) can be obtained, in an almost quantitative yield, by crystallization from either acetic anhydride/acetic acid or acetic anhydride alone, avoiding the further, complex treatment with triethylamine/acetone and reprecipitation with phosphoric acid. Instead of the latter, crystallized diacerein as above is simply washed in centrifuge with water.

When diacerein is obtained from acetylbarbaloin by chromic oxidation, any traces of chromium are removed by addition of EDTA and hot filtration of the EDTA/chromium complex, as disclosed in WO 00/68179.

According to the invention, one part by weight of crude diacerein obtained by chromic oxidation of acetylbarbaloin is dissolved with heating in 10-30 parts by volume of acetic anhydride, or in 50-100 parts by volume of an acetic anhydride/acetic acid mixture in volume ratio ranging from 10:90 to 30:70; when diacerein derives from chromic oxidation of acetylbarbaloin, ethylenediaminetetraacetic acid (EDTA) is added in amount of 0.05-0.2% by weight (on diacerein), the chromium/EDTA complex is filtered off from the hot mixture, which is then cooled to 5-25°C, the resulting precipitate is centrifuged and thoroughly washed with acetic acid, then with water. The resulting diacerein has at least 99.8% purity, and contains less than 70 p.p.m. of aloe-emodin and not more than 15 p.p.m. of chromium (atomic absorption).

The process of the invention is of course useful also to purify diacerein obtained through other procedures, which do not involve the chromic oxidation step. In this case, the addition of EDTA is superfluous.

The following examples further illustrate the process according to the invention.

### Example 1

5 Kg of crude diacerein (obtained by chromic oxidation of acetylbarbaloin, see PCT/EP/03221) are dissolved in 400 liters of a hot acetic acid/acetic anhydride 88.75/11.25 (vol/vol) mixture, 5 g of ethylenediaminetetraacetic acid are added to the solution which is filtered while hot to remove insolubles (EDTA-chromium complex), cooled at room temperature, filtered in centrifuge and washed first with acetic acid, then with water, and dried. 4.780 g of diacerein are obtained (95.6% yield) with 99.8% titre, containing < 50 p.p.m. of aloe-emodin and 13 p.p.m. of chromium.

### Example 2

The procedure of example 1 is followed, using 85 liters of acetic anhydride the crystallization solvent. Furthermore, after removing the EDTA-chromium complex, the solution is concentrated by distillation under reduced pressure to about 1/3 starting volume, then the procedure of example 1 is followed. The resulting product has the same purity characteristics as that of example 1; yield: 93%.

## Claims

1. A process for the preparation of highly pure diacerein which comprises the following steps:
a) chromic oxidation of acetylbarbaloin,
b) addition of the hot solution with 0.05 to 0.2% by weight (on diacerein) of ethylenediaminetetraacetic acid (EDTA) and filtering off from the hot mixture the EDTA-chromium, thereby yielding crude diacerein,
**characterized in that** crude diacerein is purified by crystallization from a solvent consisting of an acetic anhydride/acetic acid mixture or of acetic anhydride alone, without further treatment with triethylamine/acetone and reprecipitation with phosphoric acid.

2. A process as claimed in claim 1, wherein diacerein is dissolved in 50-100 parts by volume of an acetic anhydride/acetic acid mixture.

3. A process as claimed in claim 2, wherein the acetic anhydride/acetic acid ratio in the mixture ranges from 10:90 to 30:70.

4. A process as claimed in claim 1, wherein diacerein is dissolved in 10-30 parts by volume of acetic anhydride alone.

## Patentansprüche

1. Verfahren zur Herstellung eines hochgradig reinen Diacereins, das die nachstehenden Schritte umfasst:
a) Chromoxidation von Acetylbarbaloin,
b) Versetzen der heißen Lösung mit 0,05 bis 0,2 Gew.-% (bezüglich Diacerein) an Ethylendiamintetraessigsäure (EDTA) und Abfiltrieren des EDTA-Chroms von dem heißen Gemisch, wodurch rohes Diacerein gewonnen wird,
**dadurch gekennzeichnet, dass** rohes Diacerein durch Kristallisation aus einem Lösungsmittel, das aus einem Essigsäureanhydrid/Essigsäure-Gemisch oder aus Essigsäureanhydrid allein besteht, ohne weitere Behandlung mit Triethylamin/Aceton und erneuter Präzipitation mit Phosphorsäure aufgereinigt wird.

2. Verfahren, wie in Anspruch 1 beansprucht, wobei Diacerein in 50-100 Volumenteilen eines Essigsäureanhydrid/Essigsäure-Gemischcs gelöst wird.

3. Verfahren, wie in Anspruch 2 beansprucht, wobei das Essigsäureanhydrid/Essigsäure-Verhältnis in dem Gemisch von 10:90 bis 30:70 reicht.

4. Verfahren, wie in Anspruch 1 beansprucht, wobei Diacerein in 10-30 Volumenteilen an Essigsäureanhydrid allein gelöst wird.

## Revendications

1. Procédé pour la préparation de diacéréine extrêmement pure, qui comprend les étapes suivantes :
a) l'oxydation chromique d'acétylbarbaloin,
b) l'addition de la solution chaude en présence de 0,05 à 0,2 % en poids (sur la diacéréine) d'acide éthylènediaminetétraacétique (EDTA) et l'élimination par filtration à partir du mélange chaud de l'EDTA-chrome, en obtenant ainsi de la diacéréine brute,
**caractérisé en ce que** la diacéréine brute est purifiée par cristallisation à partir d'un solvant constitué d'un mélange d'anhydride acétique/acide acétique ou d'anhydride acétique seul, sans autre traitement avec de la triéthylamine/acétone et reprécipitation avec de l'acide phosphorique.

2. Procédé selon la revendication 1, dans lequel la diacéréine est dissoute dans 50 à 100 parties en volume d'un mélange d'anhydride acétique/acide acétique.

3. Procédé selon la revendication 2, dans lequel le rapport de l'anhydride acétique/acide acétique dans le mélange se situe entre 10:90 et 30:70.

4. Procédé selon la revendication 1, dans lequel la diacéréine est dissoute dans 10 à 30 parties en volume d'anhydride acétique seul,
